# EUROPEAN PATENT SPECIFICATION

(11) **EP 4 169 351 B1**
(45) Date of publication and mention of the grant of the patent: **24.12.2025**
(21) Application number: 21826203.8
(22) Date of filing: 08.06.2021
(51) Int. Cl.: H05B 1/02

(54) **INFANT WARMING SYSTEM AND METHOD**
SÄUGLINGSWÄRMESYSTEM UND -VERFAHREN
SYSTÈME ET PROCÉDÉ DE RÉCHAUFFEMENT DE NOURRISSONS

(30) Priority: 18.06.2020 US 202016904726
(43) Date of publication of application: 26.04.2023
(73) Proprietor: GE Precision Healthcare LLC, Wauwatosa, WI 53226 (US)
(72) Inventor: WOLOSCHEK, Steven J., Wauwatosa, Wisconsin 53226 (US); FALK, Steven M., Baltimore, Maryland 21209 (US); MEDEIROS, Daniel W., Brookfield, Wisconsin 53226 (US)
(74) Representative: Kilburn & Strode LLP
(86) International application number: PCT/US2021/036463
(87) International publication number: WO 2021/257330

(56) References cited:
- US-A1- 2005 215 844
- US-A1- 2006 049 937
- US-A1- 2008 106 421
- US-A1- 2016 302 253
- US-A1- 2017 048 651
- US-A1- 2018 049 257
- US-A1- 2019 327 774

## Description

### CROSS-REFERENCE TO RELATED APPLICATIONS

This application is a continuation of and claims priority to U.S. Patent Application Serial No. 16/904,726, filed June 18, 2020.

### BACKGROUND

The present disclosure relates generally to patient monitoring devices and systems for monitoring a patient's physiology and health status. More specifically, the present disclosure relates to infant warming systems with wireless patient monitoring devices, systems, and methods that incorporate and/or pair with wireless physiological sensors configured for measuring physiological parameter information from an infant and wirelessly transmitting that information.

In the field of medicine, physicians often desire to monitor multiple physiological characteristics of their patients. Oftentimes, patient monitoring involves the use of several separate monitoring devices simultaneously, such as an electrocardiograph (ECG), a pulse oximeter, a respiration monitor, a temperature monitor, etc. Several separate patient monitoring devices are often connected to a patient, tethering the patient to multiple bulky bedside devices via physical wiring or cables. Multi-parameter monitors are also available where different sensor sets may be connected to a single monitor. However, such multi-parameter systems may be even more restrictive than separate monitoring devices because they require all of the sensors attached to a patient to be physically attached to a single monitor, resulting in multiple wires running across the patient's body. Thus, currently available patient monitoring devices often inhibit patient movement, requiring a patient to stay in one location or to transport a large monitor with them when they move from one place to another.
US 2006/0049937 A1 discloses a care device and process. The care device has a sensor module, which is to be placed directly on the patient for picking up the patient's data, such as temperature, pulse and ECG. A first receiving means is arranged in space in the vicinity of the patient and is designed to pass on the measured data received from the sensor module. A mobile part can be removed from the care device and provides wireless communication and a second receiving means for establishing an alternative primary wireless link when the patient is removed from the care device.

### SUMMARY

The invention is set out in the appended set of claims.

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In one embodiment, an infant warming system includes a bassinet having a platform configured to support an infant, at least one wireless physiological sensor configured to measure a physiological parameter from the infant and transmit physiological parameter data, and at least two radio frequency identification (RFID) readers on the infant warming system. The RFID readers are each configured to communicate with the at least one wireless physiological sensor to facilitate pairing therewith so as to enable receipt of the physiological parameter data from the wireless physiological sensors at the infant warmer system. The RFID readers each have a range distance that is less than a length of the platform and are positioned such that the wireless physiological sensor is in the range distance of at least one of the at least two RFID readers from any location on the platform.

A bassinet configured as part of an infant warming system includes a platform configured to support an infant, at least two RFID readers on the infant care device configured to communicate with at least one wireless physiological sensor to facilitate pairing therewith so as to enable receipt of physiological parameter data from the wireless physiological sensor. The RFID readers each have a range distance that is less than a length of the platform and are positioned such that a wireless physiological sensor on an infant supported on the platform is in the range distance of at least one of the at least two RFID readers from any location on the platform.

Various other features, objects, and advantages of the invention will be made apparent from the following description taken together with the drawings.

### BRIEF DESCRIPTION OF THE DRAWINGS

The present disclosure is described with reference to the following Figures.
FIG. 1 is a schematic diagram of an infant warming system according to one embodiment of the present disclosure.
FIG. 2 depicts an infant warming system according to the present disclosure.
FIG. 3 schematically depicts a neonatal care area having three infant warming systems according to the present disclosure.
FIG. 4 depicts an embodiment of an infant warming system having four RFID readers according to one embodiment of the present disclosure.
FIG. 5 schematically depicts one embodiment of an RFID reader and sensor controller, and wireless communication therebetween.
FIGS. 6-7 depict embodiments of a method of controlling pairing and wireless communication between a wireless physiological sensor and an infant warming system.

### DETAILED DESCRIPTION

Wearable wireless physiological sensors are becoming more prevalent in several patient care and physiological monitoring areas. The inventors have recognized that one challenge to implementing wireless sensors in the area of infant care is ensuring that patient monitoring data wirelessly transmitted is received and correctly associated with the patient. In neonatal care environments, such as neonatal care units (NICU), multiple patients are often cared for in a relatively small environment. Having multiple neonates wearing wireless physiological sensors and multiple receiving devices, e.g., multiple warming systems, in a small area increases the opportunity and likelihood of stray reads, where physiological data transmitted by a sensor on one infant is received by a receiving device associated with another infant. If warming systems are not properly paired, patient monitoring data transmitted by wireless sensors may be misassociated with the wrong neonate, which can cause confusion, delay in care, or worse.

Thus, accurate pairing between wireless sensors and patient monitoring devices is highly important. However, current pairing methods for wireless patient monitoring are cumbersome and error-prone. Typically, sensors need to be manually paired with a patient monitoring system or other receiving system via a user interface or by swiping the sensor near a wireless receiver, such as a near field communication (NFC) reader to initiate the pairing process. Labor and delivery and NICU environments present particularly challenging environments in that they are often crowded and hectic where caregivers are caring for multiple patients at any given time. Manual pairing requires the caregiver to stop patient care and pair a sensor before placing it on the infant. This is a distraction from critical care. Moreover, where multiple patients are within range of the wireless sensor and communication protocols are utilized that allow pairing from a distance, the opportunity for stray reads and improper pairing presents itself.

In view of the foregoing challenges and problems in the relevant art, the inventors have developed the disclosed system and method which allow pairing between receiving devices and wireless sensors and allow reading of RFID tags on the wireless sensors only in a limited area encompassing the infant care device, such as the incubator or warmer, in which the infant is housed. The disclosed system and method allows the clinician to simply attach a wireless physiological sensor to an infant housed in an infant warming system, where the infant warming system is configured to automatically detect and facilitate pairing with that wireless physiological sensor. In one embodiment, the infant warming system has multiple radio frequency identification (RFID) readers in the bassinet housing the infant, where the RFID readers are positioned such that each of the at least one wireless physiological sensors is in a range distance of at least one of the RFID readers for purposes of pairing from any location on the platform. In one embodiment, the RFID readers are NFC readers and configured to exchange information to execute at least a portion of the pairing, such as to exchange encrypted pairing information, via NFC such that the system can automatically enable pairing between the wireless physiological sensor and the infant warming system. In certain embodiments, completion of the pairing process and communication of physiological information between the infant warming system and wireless physiological sensor may be executed through a different wireless communication protocol than the pairing initiation executed by the above-mentioned RFID readers on the bassinet, such as Bluetooth.

In one embodiment, a bassinet having a platform configured to support an infant includes at least two RFID readers on the infant care device on or around the platform, where the RFID readers each have a range distance for purposes of pairing that is less than a length of a platform. The at least two RFID readers are positioned such that each wireless physiological sensor on an infant in the bassinet is in a range distance of at least one of the two RFID readers from any location on the platform. For instance, the RFID readers may be high-frequency RFID (HF RFID) readers, such as NFC circuits, positioned such that each wireless physiological sensor on an infant housed in the infant warming system is within the range distance of at least one of the HF RFID reader from any location on the platform, wherein the HF RFID readers are positioned to minimize overlap of the ranges.

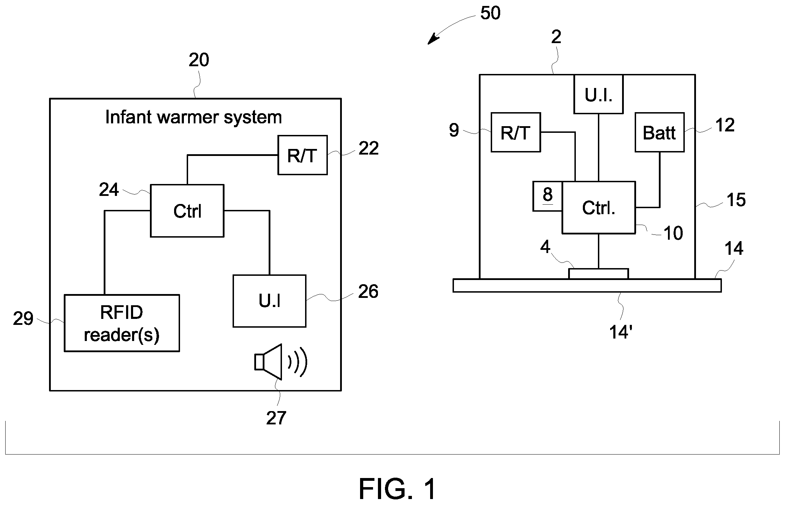

FIG. 1 depicts one embodiment of a wireless patient monitoring system 50 configured to monitor one or more physiological parameters of a patient, and particularly an infant. The patient monitoring system 50 is incorporated in an infant warming system 20, such as an incubator or warmer system. The patient monitoring system 50 includes a wireless physiological sensor 2 configured to communicate with a controller 24, which is a controller configure to facilitate physiological monitoring of the infant. The wireless physiological sensor 2 has a sensing element 4 arranged on a substrate 14. The sensor controller 10 receives physiological information detected by the sensing element 4. The sensing element 4 may be any type of device for sensing or detecting physiological information from the patient, which may include but is not limited to a skin electrode, temperature sensor, pressure sensor, flow sensor, infrared or other pulse oximetry sensor, or the like. For instance, the monitored parameter value may be heart rate, respiration rate, SpO2, or temperature. The wireless physiological sensor 2 shown in FIG. 1 includes a sensor module 15 mounted on the substrate 14 and housing the sensor controller 10, a first transmitter 9 (which may be a transceiver), a second transmitter 8 configured for communication via a different wireless protocol than the first transmitter, and a battery 12 to power the wireless sensor. The sensor module 15 may comprise a housing that is attached to the substrate 14 and configured to house and protect the various components of the sensor 2.

The sensor controller 10 is configured to receive and process the physiological information from the sensing element 4, such as to filter and digitize the information, as well as to process the digital signal to extract relevant physiological values therefrom. The sensor controller 10 may include a processor as well as signal processing elements, including filters, amplifiers, or the like as is required or appropriate for processing the type of physiological information that the sensing element 4 is configured to detect. In certain types of physiological sensors 2, the sensor controller 10 may be configured to determine a discrete value based on the physiological parameter information received from the sensing element 4, such as a heart rate, respiration rate, SpO2, temperature, etc.

A wireless transmitter 9 (which may also be a transmitter/receiver or transceiver) communicates the recorded physiological parameter values and other information to the infant warming system 20, such as a patient monitoring subsystem incorporated therein and configured to receive the physiological measurements. The transmitter 9 is configured to communicate the physiological information to the infant warming system 20 by a wireless communication means, which may include any appropriate wireless communication protocol. In one embodiment, the infant warming system 20 is also configured to communicate information to the sensor, and thus is configured with a transceiver 22 that communicates with a transceiver 9 in the physiological sensor 2. In one embodiment, the transceiver 22 is configured as a body area network with one or more transceivers 9 in one or more physiological sensors 2 on the patient. In other embodiments, the physiological sensor 2 and infant warming system 20 may communicate by other radio protocols, such as but not limited to Bluetooth, Bluetooth Low Energy (BLE), ANT, and Zigbee.

The sensor 2 and infant warming system 20 may be configured to utilize a different wireless protocol for pairing than for transmission of physiological data, where the pairing protocol is preferably a wireless protocol requiring close-range communication such as near field communication (NFC). Thereby, problems of mispairing based on stray reads can be mitigated or avoided entirely. Accordingly, the infant warming system may include one or more RFID readers 29 configured for RFID communication and having a close communication range distance-e.g., 10 cm where NFC is utilized or other ranges due to HF RFID configured with a short-range distance (e.g., with a maximum range distance between 5 cm and 20 cm).

Thereby, the RFID readers 29 are only capable of pairing communication with wireless physiological sensors 2 located on an infant within that infant warmer system 20 and not on an infant in another warmer system nearby. Moreover, the system may be configured to easily and automatically facilitate pairing between a wireless sensor 2 and the infant warming system 20, where the system 20 is configured to detect a new sensor on the infant 1 housed therein. Given the short detection range of the RFID readers 29, identification of the sensors only on the infant 1 housed in the system 20 can be guaranteed. This can facilitate an easy pairing operation for new sensors, where a clinician simply puts the new sensor on the infant and the system 20 is configured to automatically initiate and facilitate pairing. Similarly, the system 20 may be configured to automatically detect and pair with all sensors on an infant 1 when that infant is placed in the warming system 20.

The wireless physiological sensor 2 includes an RFID transmitter 8, which may be an RFID transceiver, configured to communicate with the one or more RFID readers 29 positioned in the warmer system 20, such as on or around the platform 112. For example, the RFID transmitter 8 may be an NFC transmitter, such as an NFC circuit. In other embodiments, the RFID transmitter 8 may be configured for communication via a different protocol, such as via HF RFID and configured for communication only over a short-range distance. The RFID transmitter 8 may be a separate device from the receiver/transmitter 9, where the RFID transmitter 8 is configured for shorter range communication and the transceiver 9 is configured for longer-range communication. In other embodiments, one radio communication system may be provided and capable of communicating via two different protocols-e.g., a shorter range protocol for pairing (or at least a portion of the pairing process) and a longer range protocol for communicating physiological parameter data etc.

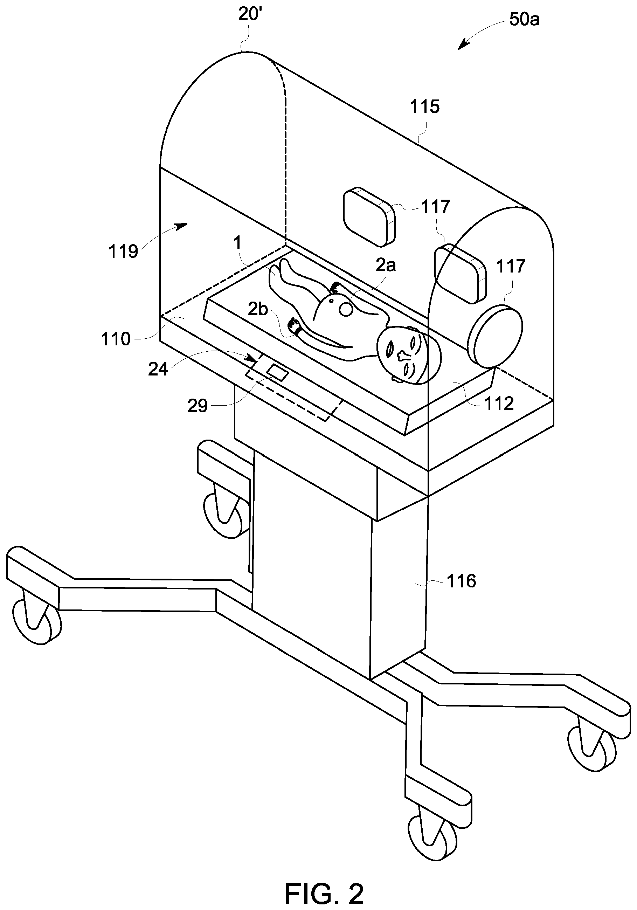

FIG. 2 depicts an exemplary wireless physiological monitoring arrangement in an infant warming system 20, which in the depicted example is an incubator 20'. In the depicted example, the physiological monitoring system 50 is temperature monitoring system 50a, but a person of ordinary skill in the art will understand that the disclosed method and system may encompass any type of physiological system for a neonate housed in an infant warming system 20. The temperature monitoring system 50a includes two wireless temperature sensors 2a and 2b attached to the neonate to determine neonatal temperature. The first temperature sensor 2a senses a body temperature of the neonate and the second temperature sensor 2b senses a peripheral temperature. The temperature sensors 2a and 2b are utilized to monitor and maintain an appropriate environment for the neonate 1. The incubator 20', which in other embodiments could be another type of infant warming device 20 such as a radiant warmer, has a heater system providing a heated environment for the infant 1. The controller 24 includes software that processes the measurements from the respective temperature probes 2a, 2b to control various aspects of the system, including the heater.

In the exemplary incubator system of FIG. 2, the incubator 20' includes a bassinet 110 having a platform 112 supporting the infant 1. In the depicted example, the platform 112 is a mattress or other flat surface supporting the infant 1 and located on the bassinet 110. In some embodiments, the bassinet 110 may have vertical sidewalls (not shown) that extend upward around the platform 112.A canopy 115 is provided that is positioned atop the bassinet 110 and provides a covered area over the platform 112 so as to enclose the infant and form a microenvironmental chamber 119 providing a controlled environment isolated from the surrounding environment. The canopy 115 has access portals 117 to facilitate access to the infant 1 without significantly altering the microenvironment within the chamber 119. The canopy 115 is supported on a support structure 116, or frame, that houses and supports control systems for controlling aspects of the microenvironment within the chamber 119, including a heater system, as well as other systems for controlling humidity, airflow, etc. within the chamber 119. In other embodiments a radiant warmer may be located above the bassinet 110 and controllable to heat the environment in and around the bassinet 110 housing the infant.

In the examples, the system 20 includes a body temperature probe 2a removably fixed to the infant's torso, such as to the infant's abdomen, to measure a body temperature of the infant 1, and includes a peripheral temperature probe 2b removably fixed to the infant's extremity to measure a peripheral temperature of the infant 1. Each temperature probe 2a, 2b has a respective temperature sensing element thermally contacting and detecting a temperature at a particular location on the infant's skin. In the particular embodiment, the body temperature probe 2a comprises an adhesive connection on the bottom side 14' of the substrate 14 adhering the wireless body temperature sensor 2a to the skin of an infant's torso, such as above the infant's liver. In the depicted embodiment, the peripheral temperature probe 2b has a fixation band fixing the peripheral temperature sensor 2b to the infant's hand.

The incubator 20' contains one or more RFID readers 29 configured to communicate with RFID transmitters 8 in the wireless sensors (e.g., 2a and 2b) for purposes of recognizing sensors 2 and/or for pairing. For example, each RFID reader 29 may be an NFC circuit 29 that emits an NFC field. The temperature sensors 2a and 2b each incorporate an NFC circuit as the RFID transmitter 8, which each emit their own field. Thus, when one of the sensors 2a, 2b are in within range of the NFC field when the sensor 2a, 2b is in close proximity to the NFC circuit 29. Pairing between each temperature sensor 2a, 2b and the host controller 24 of the incubator 20' is then executed. Once pairing occurs, the temperature of the microenvironment 119 maintained in the incubator 20' can be controlled based on the infant's temperature.

The infant warming system 20 may include a host controller 24, which may be configured to process and/or display physiological data recorded by the sensors 2 (e.g. temperature sensors 2a and 2b). The infant warming system 20 may include a user interface 26, such as for displaying the physiological information recorded by the sensor 2. The user interface may include a display device and may also include one or more speakers 27 or buzzers for generating an audio alert. The user interface 26 may further be configured to facilitate pairing between the infant warming system 20 and the sensors 2. For example, the user interface 26 may be configured to collect user approval or instruction for pairing. For example, the controller 24 may operate the user interface 26 to display a list of all wireless sensors 2 within range of all of the RFID readers 29 in the infant warming device 20. The user interface 26 may be configured to receive a user selection input from a user to select one of the physiological sensors for pairing.

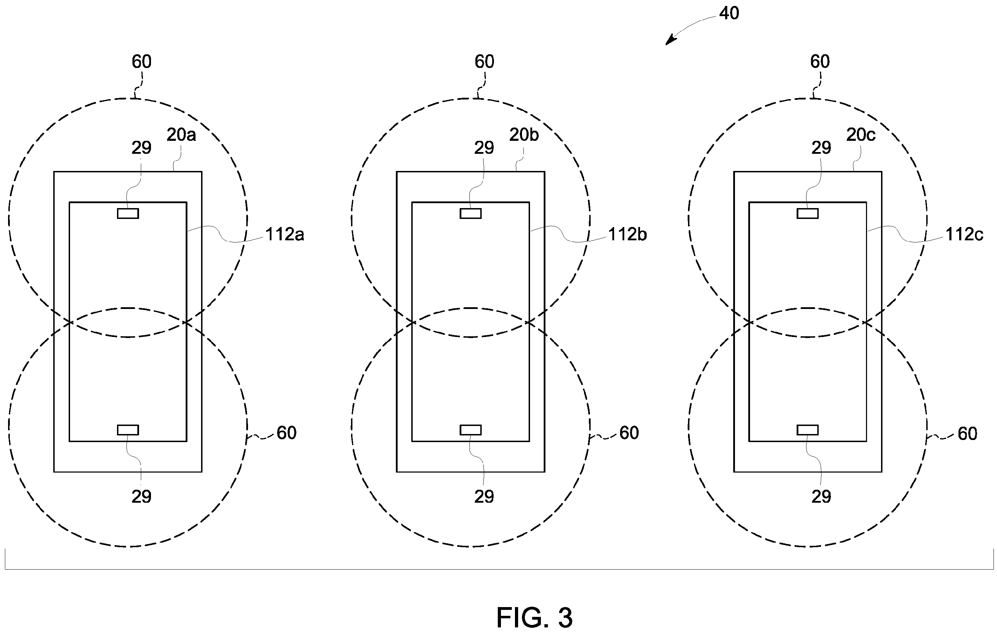

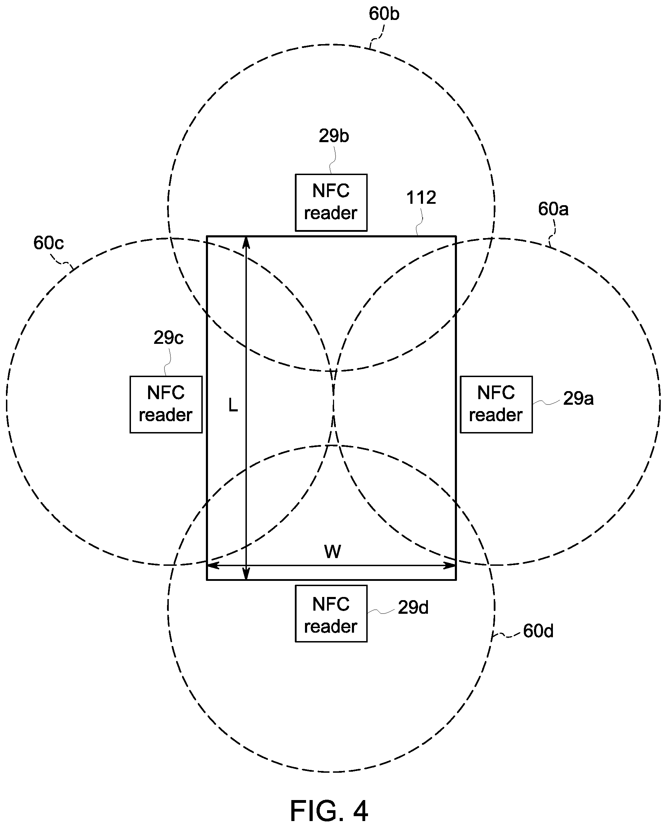

In one embodiment, the range distance of communication between the wireless physiological sensor 2 and infant warming system 20 for purposes of pairing is less than the length of a platform 112 in the infant warming system 20. In another embodiment, the range distance of communication between the wireless physiological sensor 2 and infant warming system 20 for purposes of pairing is less than the width of the platform 112 in the infant warming system 20. FIGS. 3 and 4 depict exemplary RFID receiver arrangements, where the RFID readers 29 are arranged to provide receiver coverage over the entire platform 112 for purposes of sensor detection and pairing. The RFID readers 29 may be configured and positioned to minimize overlap between the reader ranges, such as in the embodiments shown where the ranges are sized and positioned such that overlap is minimized while complete coverage of the platform 112 is accomplished.

FIG. 3 depicts an embodiment of a neonatal care area 40 housing three infant warming systems 20a-20c, each configured to house an infant 1 to which wireless physiological sensors are attached. Each of the infant warming systems 20a-20c includes two RFID readers 29, wherein each RFID reader 29 has a range distance 60 that is less than a length of the platform 112. With reference also to FIG. 4, the platform 112 has a length L and a width W. The platform 112 is generally configured to support the infant 1 arranged from head to toe along the length L of the platform 112. The range distances 60 are sufficient such that a sensor at any location on the platform 112 will be in range of at least one of the two RFID readers. However, the range distances 60 are small enough that they will not cross into an area occupied by an adjacent infant warming system 20. Thus, a wireless physiological sensor on an infant in a second, adjacent infant warming system will not be picked up by one of the RFID readers 29 in a first warming system. Thereby, mispairing and stray reads can be avoided.

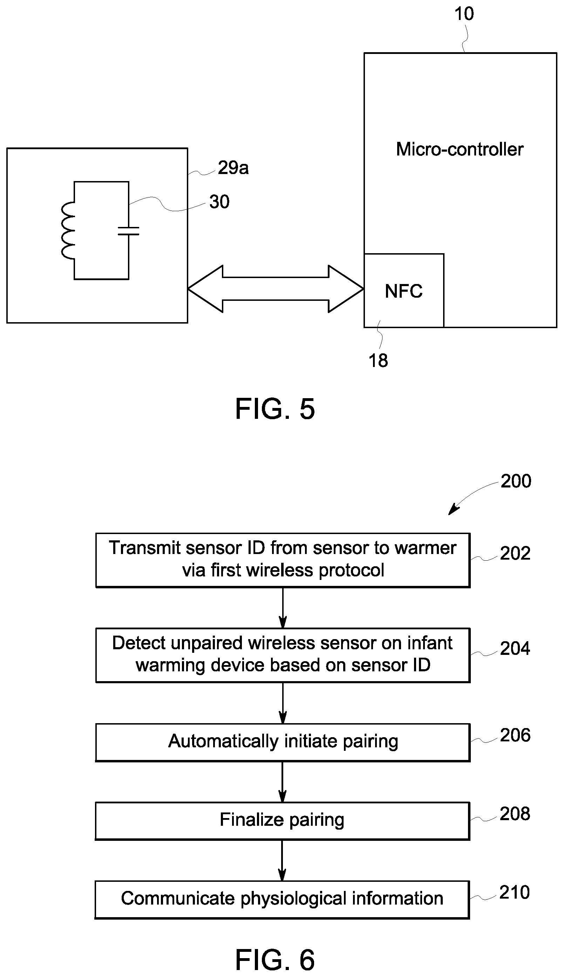

FIG. 4 depicts another embodiment of an infant warming system 20 having four RFID readers 29, which in the depicted embodiment are NFC readers 29a-29d. In this embodiment, the four NFC readers 29a-29d are arranged one on each of the four sides of the platform 112. Here, the range distance 60a-60d of each of the NFC readers 29a-29d is less than a width W of the platform 112. For example, the readers 29a-29d pay be placed on a flat surface parallel to the surface of the platform 112 or may be on a surface perpendicular to the platform 112, such as on a vertical wall of the bassinet 110 surrounding the platform 112. In some embodiments, the range distances 60a-60d may be equal, in others one or more of the range distances 60a-60d may be configured to be greater or less than others so that full range coverage is provided over the platform 112 while overlap is minimized. FIG. 5 depicts one exemplary NFC arrangement, which includes an NFC reader 29a being an NFC circuit having an NFC antenna 30. The transmitter 8 in the sensor 2 may be, for example, an NFC peripheral 18 incorporated into the sensor controller 10 or may be a standalone NFC circuit in communication with the sensor controller 10.

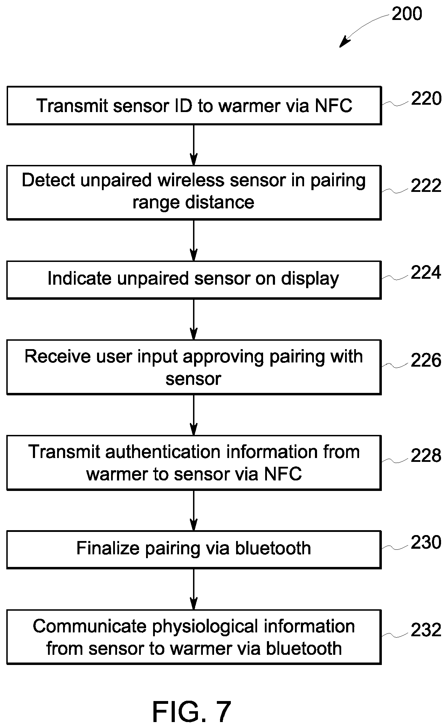

FIGS. 6 and 7 depict embodiments of methods 200 of controlling a patient monitoring system 50 that is part of an infant warmer system 20, and more particularly for controlling pairing between a wireless physiological sensor 2 and a patient monitoring system incorporated in the infant warmer system 20. Referring to FIG. 6, a sensor identification (ID) is transmitted from a wireless physiological sensor 2 to an infant warmer system 20 at step 202 via a first wireless protocol. Preferably, the wireless communication and protocol are configured for close-range wireless communication, such as a range that is contained within the area of the infant warmer system 20 only. An unpaired wireless system is detected at the infant warming device at step 204 based on the transmitted sensor ID. Pairing is automatically initiated at step 206. For example, the RFID reader 29a and RFID transmitter 8 may be configured to set up authentication for transmission via NFC. For instance, the RFID reader 29 may transmit a warmer system ID to the sensor and/or may transmit encryption information to initiate pairing via the first protocol, which is the close-range protocol. Pairing is then finalized at step 208. In certain embodiments, the remainder of the pairing steps may be executed via a second protocol, which may be a longer range protocol. For instance, the first wireless protocol may be NFC and the second wireless protocol may be Bluetooth, just to provide one example. Once pairing is complete, physiological information is then communicated at step 210 from the wireless physiological sensor 2 to the infant warming system 20.

FIG. 7 depicts another embodiment of method 200 of facilitating pairing. The sensor ID is transmitted to the warmer system at step 220 via NFC. The warmer system detects an unpaired wireless sensor within pairing range distance at step 222. The warmer system then indicates an unpaired sensor on a display associated with the warmer at step 224. For example, the user interface 26 may be controlled to display a list of wireless physiological sensors within range of the at least one RFID readers 29, such as an unpaired sensor within range. User input may then be received at step 226 approving pairing with the detected sensor. Pairing between the sensor and warmer device is then facilitated. For example, authentication information may be transmitted from the warmer to the sensor via NFC at step 228. Pairing may then be finalized via a different wireless communication protocol, such as Bluetooth, at step 230. After completion of the pairing, the physiological information may be communicated from the wireless physiological sensor 2 to the infant warming device 20 via the same longer-range protocol, such as Bluetooth, as represented at step 232.

This written description uses examples to disclose the invention, including the best mode, and also to enable any person skilled in the art to make and use the invention. Certain terms have been used for brevity, clarity and understanding. No unnecessary limitations are to be inferred therefrom beyond the requirement of the prior art because such terms are used for descriptive purposes only and are intended to be broadly construed. The patentable scope of the invention is defined by the claims.

## Claims

1. An infant warming system (20) comprising:
a platform (112) configured to support an infant (1);
at least one wireless physiological sensor (2a, 2b) configured to measure a physiological parameter from the infant and transmit physiological parameter data;
at least two radio frequency identification (RFID) readers (29) configured to communicate with the at least one wireless physiological sensor to facilitate pairing therewith so as to enable receipt of the physiological parameter data therefrom;
wherein the RFID readers each have a range distance that is less than a length of the platform and the at least two RFID readers are positioned such that each of the at least one wireless physiological sensor is in the range distance of at least one of the at least two RFID readers from any location on the platform.

2. The infant warming system of claim 1, wherein the RFID readers are configured as high frequency RFID (HF RFID) readers.

3. The infant warming system of claim 2, further comprising at least three HF RFID readers positioned such that each of the at least one wireless physiological sensor is in the range distance of at least one of the at least three HF RFID readers from any location on the platform.

4. The infant warming system of claim 2, at least four HF RFID readers, each with a range distance less than a width of the platform and positioned such that each of the at least one wireless physiological sensor is in the range distance of at least one of the at least four RFID readers from any location on the platform and to minimize overlap of the distance ranges.

5. The infant warming system of claim 4, wherein the HF RFID readers are NFC devices and the range distance is 10 cm or less.

6. The infant warming system of claim 1, further comprising a controller (24) configured to control pairing between each of the at least one wireless physiological sensor and all of the RFID readers.

7. The infant warming system of claim 6, further comprising a user interface (26) on the infant warming system, wherein the controller is configured to control the user interface to require user input to approve the pairing between each of the wireless physiological sensors and all of the RFID readers.

8. The infant warming system of claim 7, wherein the user interface (26) is configured to display a list of one or more wireless physiological sensors within the distance range of at least one of the RFID readers.

9. The infant warming system of claim 8, wherein the user interface is configured to receive a user selection at the user interface of a selected physiological sensor from the list of wireless physiological sensors and the controller is configured to then pair with the selected wireless physiological sensor.

10. The infant warming system of claim 6, wherein the controller is configured to automatically initiate pairing with each of the at least one wireless physiological sensor upon receipt of a sensor identification from an unpaired sensor within the distance range of one of the at least two RFID readers.

11. The infant warming system of claim 1, further comprising at least one NFC circuit on the infant warming system, wherein pairing between the wireless physiological sensor and the infant warming system is initiated when the wireless physiological sensor enters the range distance of the NFC circuit.

12. The infant warming system of claim 11, wherein the wireless physiological sensor further includes an NFC circuit and is configured to only transmit its sensor identification to the infant warming system via NFC.

13. The infant warming system of claim 1, wherein the infant warming system is an incubator or a radiant warmer.

14. A bassinet configured as part of an infant warming system, the bassinet comprising:
a platform (112) configured to support an infant;
at least two radio frequency identification (RFID) readers (29) on or around the platform configured to communicate with at least one wireless physiological sensor to facilitate pairing therewith so as to enable receipt of physiological parameter data therefrom; and
wherein the RFID readers each have a range distance that is less than a length of the platform and the at least two RFID readers are positioned such that a wireless physiological sensor is in the range distance of at least one of the at least two RFID readers from any location on the platform.

15. The bassinet of claim 14, further comprising at least one NFC circuit on the bassinet, wherein pairing between the wireless physiological sensor and the infant warming system is initiated when the wireless physiological sensor enters the range distance of the NFC circuit.

## Patentansprüche

1. Säuglingswärmesystem (20), das Folgendes umfasst:
eine Plattform (112), die konfiguriert ist, einen Säugling (1) zu tragen;
mindestens einen drahtlosen physiologischen Sensor (2a, 2b), der konfiguriert ist, einen physiologischen Parameter von dem Säugling zu messen und physiologische Parameterdaten zu übertragen;
mindestens zwei Hochfrequenz-Identifikationslesegeräte (RFID-Lesegeräte) (29), die konfiguriert sind, mit dem mindestens einen drahtlosen physiologischen Sensor zu kommunizieren, um die Kopplung damit zu ermöglichen, um den Empfang der physiologischen Parameterdaten davon zu aktivieren;
wobei die RFID-Lesegeräte jeweils einen Reichweitenabstand aufweisen, der kleiner ist als eine Länge der Plattform, und die mindestens zwei RFID-Lesegeräte derart positioniert sind, dass sich jeder des mindestens einen physiologischen Sensors von jedem beliebigen Ort auf der Plattform in dem Reichweitenabstand von mindestens einem der mindestens zwei RFID-Lesegeräte befindet.

2. Säuglingswärmesystem nach Anspruch 1, wobei die RFID-Lesegeräte als Hochfrequenz-RFID-Lesegeräte (HF-RFID-Lesegeräte) konfiguriert sind.

3. Säuglingswärmesystem nach Anspruch 2, das ferner mindestens drei HF-RFID-Lesegeräte umfasst, die derart positioniert sind, dass sich jeder des mindestens einen physiologischen Sensors von einem beliebigen Ort auf der Plattform in dem Reichweitenabstand von mindestens einem der mindestens drei HF-RFID-Lesegeräte befindet.

4. Säuglingswärmesystem nach Anspruch 2, das ferner mindestens vier HF-RFID-Lesegeräte umfasst, die jeweils einen Reichweitenabstand aufweisen, der kleiner ist als eine Breite der Plattform, und derart positioniert sind, dass sich jeder des mindestens einen drahtlosen physiologischen Sensors von jedem Ort auf der Plattform in dem Reichweitenabstand von mindestens einem der mindestens vier RFID-Lesegeräte befindet und dass die Überlagerung der Reichweitenabstände minimiert wird.

5. Säuglingswärmesystem nach Anspruch 4, wobei die HF-RFID-Lesegeräte NFC-Vorrichtungen sind und der Reichweitenabstand 10 cm oder weniger beträgt.

6. Säuglingswärmesystem nach Anspruch 1, das ferner eine Steuereinrichtung (24) umfasst, die konfiguriert ist, die Kopplung zwischen jedem des mindestens einen drahtlosen physiologischen Sensors und allen RFID-Lesegeräten zu steuern.

7. Säuglingswärmesystem nach Anspruch 6, das ferner eine Benutzerschnittstelle (26) an dem Säuglingswärmesystem umfasst, wobei die Steuereinrichtung konfiguriert ist, die Benutzerschnittstelle zu steuern, um eine Benutzereingabe anzufordern, um die Kopplung zwischen jedem der drahtlosen physiologischen Sensoren und allen RFID-Lesegeräten zu genehmigen.

8. Säuglingswärmesystem nach Anspruch 7, wobei die Benutzerschnittstelle (26) konfiguriert ist, eine Liste von einem oder mehreren drahtlosen physiologischen Sensoren in dem Reichweitenabstand des mindestens einen der RFID-Lesegeräte anzuzeigen.

9. Säuglingswärmesystem nach Anspruch 8, wobei die Benutzerschnittstelle konfiguriert ist, an der Benutzerschnittstelle eine Benutzerauswahl eines ausgewählten physiologischen Sensors aus der Liste von drahtlosen physiologischen Sensoren zu empfangen, und die Steuereinrichtung konfiguriert ist, anschließend mit dem ausgewählten drahtlosen physiologischen Sensor zu koppeln.

10. Säuglingswärmesystem nach Anspruch 6, wobei die Steuereinrichtung konfiguriert ist, bei Empfang einer Sensoridentifikation von einem ungekoppelten Sensor in dem Reichweitenabstand von einem der mindestens zwei RFID-Lesegeräte die Kopplung mit jedem des mindestens einen drahtlosen physiologischen Sensors automatisch zu initiieren.

11. Säuglingswärmesystem nach Anspruch 1, das ferner mindestens eine NFC-Schaltung an dem Säuglingswärmesystem umfasst, wobei die Kopplung zwischen dem drahtlosen physiologischen Sensor und dem Säuglingswärmesystem initiiert wird, wenn der drahtlose physiologische Sensor in den Reichweitenabstand der NFC-Schaltung eintritt.

12. Säuglingswärmesystem nach Anspruch 11, wobei der drahtlose physiologische Sensor ferner eine NFC-Schaltung enthält und konfiguriert ist, nur seine Sensoridentifikation über NFC zu dem Säuglingswärmesystem zu übertragen.

13. Säuglingswärmesystem nach Anspruch 1, wobei das Säuglingswärmesystem ein Inkubator oder ein Strahlungswärmer ist.

14. Stubenwagen, der als Teil eines Säuglingswärmesystems konfiguriert ist, wobei der Stubenwagen Folgendes umfasst:
eine Plattform (112), die konfiguriert ist, einen Säugling zu tragen;
mindestens zwei Hochfrequenz-Identifikationslesegeräte (RFID-Lesegeräte) (29) an der oder um die Plattform, die konfiguriert sind, mit mindestens einem drahtlosen physiologischen Sensor zu kommunizieren, um die Kopplung damit zu ermöglichen, um den Empfang seiner physiologischen Parameterdaten zu aktivieren; und
wobei die RFID-Lesegeräte jeweils einen Reichweitenabstand aufweisen, der kleiner ist als eine Länge der Plattform, und die mindestens zwei RFID-Lesegeräte derart positioniert sind, dass sich ein drahtloser physiologischer Sensor von jedem beliebigen Ort auf der Plattform in dem Reichweitenabstand von mindestens einem der mindestens zwei RFID-Lesegeräte befindet.

15. Stubenwagen nach Anspruch 14, der ferner mindestens eine NFC-Schaltung an dem Stubenwagen umfasst, wobei die Kopplung zwischen dem drahtlosen physiologischen Sensor und dem Säuglingswärmesystem initiiert wird, wenn der drahtlose physiologische Sensor in den Reichweitenabstand der NFC-Schaltung eintritt.

## Revendications

1. Système (20) de réchauffement de nourrissons, comprenant :
une plate-forme (112), configurée pour supporter un nourrisson (1) ;
au moins un capteur physiologique sans fil (2a, 2b), configuré pour mesurer un paramètre physiologique du nourrisson et transmettre des données de paramètres physiologiques ;
au moins deux lecteurs (29) d'identification par radiofréquence (RFID), configurés pour communiquer avec ledit au moins un capteur physiologique sans fil afin de faciliter l'appairage avec celui-ci de manière à permettre la réception des données de paramètres physiologiques provenant de celui-ci ;
les lecteurs RFID ayant chacun une portée inférieure à la longueur de la plate-forme et lesdits au moins deux lecteurs RFID étant positionnés de telle sorte que chaque capteur parmi ledit au moins un capteur physiologique sans fil se trouve dans la portée d'au moins un parmi lesdits au moins deux lecteurs RFID depuis n'importe quel emplacement sur la plate-forme.

2. Système de réchauffement de nourrissons selon la revendication 1, dans lequel les lecteurs RFID sont configurés en tant que lecteurs RFID haute fréquence (HF RFID).

3. Système de réchauffement de nourrissons selon la revendication 2, comprenant en outre au moins trois lecteurs RFID HF positionnés de telle sorte que chaque capteur parmi ledit au moins un capteur physiologique sans fil se trouve dans la portée d'au moins un parmi lesdits au moins trois lecteurs RFID HF depuis n'importe quel emplacement sur la plate-forme.

4. Système de réchauffement de nourrissons selon la revendication 2, comprenant au moins quatre lecteurs RFID HF, chacun ayant une portée inférieure à la largeur de la plate-forme et positionné de telle sorte que chaque capteur parmi ledit au moins un capteur physiologique sans fil se trouve dans la portée d'au moins un parmi lesdits au moins quatre lecteurs RFID depuis n'importe quel emplacement sur la plate-forme et afin de minimiser le chevauchement des portées.

5. Système de réchauffement de nourrissons selon la revendication 4, dans lequel les lecteurs RFID HF sont des dispositifs NFC et la portée est de 10 cm ou moins.

6. Système de réchauffement de nourrissons selon la revendication 1, comprenant en outre un dispositif de commande (24) configuré pour commander l'appairage entre chaque capteur parmi ledit au moins un capteur physiologique sans fil et tous les lecteurs RFID.

7. Système de réchauffement de nourrissons selon la revendication 6, comprenant en outre une interface utilisateur (26) sur le système de réchauffement de nourrissons, le dispositif de commande étant configuré pour commander à l'interface utilisateur de demander à l'utilisateur d'approuver l'appairage entre chaque capteur parmi les capteurs physiologiques sans fil et tous les lecteurs RFID.

8. Système de réchauffement de nourrissons selon la revendication 7, dans lequel l'interface utilisateur (26) est configurée pour afficher une liste d'un ou plusieurs capteurs physiologiques sans fil se trouvant dans la portée d'au moins un des lecteurs RFID.

9. Système de réchauffement de nourrissons selon la revendication 8, dans lequel l'interface utilisateur est configurée pour recevoir une sélection par l'utilisateur opérée sur l'interface utilisateur d'un capteur physiologique sélectionné dans la liste des capteurs physiologiques sans fil et le dispositif de commande est configuré pour s'appairer ensuite avec le capteur physiologique sans fil sélectionné.

10. Système de réchauffement de nourrissons selon la revendication 6, dans lequel le dispositif de commande est configuré pour initier automatiquement l'appairage avec chaque capteur parmi ledit au moins un capteur physiologique sans fil à la réception d'une identification de capteur provenant d'un capteur non appairé se trouvant dans la portée d'un des au moins deux lecteurs RFID.

11. Système de réchauffement de nourrissons selon la revendication 1, comprenant en outre au moins un circuit NFC sur le système de réchauffement de nourrissons, l'appairage entre le capteur physiologique sans fil et le système de réchauffement de nourrissons étant initié lorsque le capteur physiologique sans fil entre dans la portée du circuit NFC.

12. Système de réchauffement de nourrissons selon la revendication 11, dans lequel le capteur physiologique sans fil comprend en outre un circuit NFC et est configuré pour transmettre uniquement son identification de capteur au système de réchauffement de nourrissons via le NFC.

13. Système de réchauffement de nourrissons selon la revendication 1, dans lequel le système de réchauffement de nourrissons est une couveuse ou un radiateur rayonnant.

14. Berceau configuré comme partie intégrante d'un système de réchauffement de nourrissons, le berceau comprenant :
une plate-forme (112) configurée pour supporter un nourrisson ;
au moins deux lecteurs (29) d'identification par radiofréquence (RFID) sur ou autour de la plate-forme, configurés pour communiquer avec au moins un capteur physiologique sans fil afin de faciliter l'appairage avec celui-ci de manière à permettre la réception de données de paramètres physiologiques provenant de celui-ci ; et
dans lequel les lecteurs RFID ont chacun une portée inférieure à la longueur de la plate-forme et lesdits au moins deux lecteurs RFID sont positionnés de telle sorte qu'un capteur physiologique sans fil se trouve dans la portée d'au moins un desdits au moins deux lecteurs RFID depuis n'importe quel emplacement sur la plate-forme.

15. Berceau selon la revendication 14, comprenant en outre au moins un circuit NFC sur le berceau, l'appairage entre le capteur physiologique sans fil et le système de réchauffement de nourrissons étant initié lorsque le capteur physiologique sans fil entre dans la portée du circuit NFC.
